(19) Europäisches Patentamt
European Patent Office
Office européen des brevets

(11) Veröffentlichungsnummer: **0 250 357**
**A1**

(12) # EUROPÄISCHE PATENTANMELDUNG

(21) Anmeldenummer: **87730064.0**

(22) Anmeldetag: **12.06.87**

(51) Int. Cl.[4]: **C 07 D 457/12**
A 61 K 31/48

(30) Priorität: **16.06.86 DE 3620293**

(43) Veröffentlichungstag der Anmeldung:
**23.12.87 Patentblatt 87/52**

(84) Benannte Vertragsstaaten:
**CH DE FR GB IT LI**

(71) Anmelder: **SCHERING AKTIENGESELLSCHAFT Berlin und Bergkamen**
**Müllerstrasse 170/178 Postfach 65 03 11**
**D-1000 Berlin 65 (DE)**

(72) Erfinder: **Sauer, Gerhard, Dr.**
**Königsbacher Zeile 41 A**
**D-1000 Berlin 28 (DE)**

**Schröter, Bernd**
**Kopenhagener Strasse 28**
**D-1000 Berlin 51 (DE)**

**Wachtel, Helmut, Dr.**
**Suaretzstrasse 22**
**D-1000 Berlin 19 (DE)**

(54) **1- und/oder 2-substituierte Ergolinderivate.**

(57) Verbindungen der allgemeinen Formel I

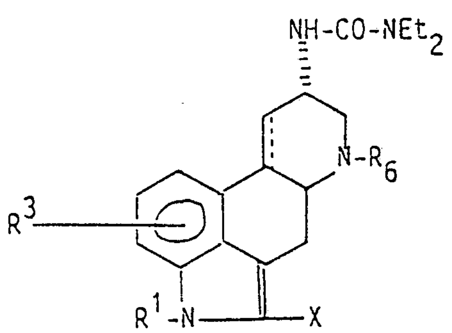

(I),

worin
X eine $CH_2$-$R^2$-Gruppe darstellt mit $R^2$ in der Bedeutung einer nucleophilen Gruppe und
$R^1$ Wasserstoff, niederes Alkyl oder X in der obengenannten Bedeutung ist, und
$R^3$ Wasserstoff oder Brom ist und
$R^6$ niederes Alkyl ist und
$C_9$---$C_{10}$ eine CC-Einfach-oder CC-Doppelbindung bedeutet,

sowie deren Säure additionssalze, ihre Herstellung und ihre Verwendung als Arzneimittel.

EP 0 250 357 A1

**Beschreibung**

1-und/oder 2-substituierte Ergolinderivate

Die Erfindung betrifft 1-und/oder 2-substituierte Ergolinylharnstoffderivate, ihre Herstellung und ihre Verwendung als Arzneimittel.

Die erfindungsgemäßen Verbindungen haben die allgemeine Formel I

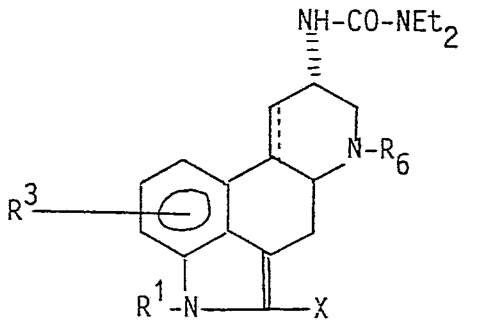

(I),

worin

X eine $CH_2$-$R^2$-Gruppe darstellt mit $R^2$ in der Bedeutung einer nucleophilen Gruppe und

$R^1$ Wasserstoff, niederes Alkyl oder X in der obengenannten Bedeutung ist, und

$R^3$ Wasserstoff oder Brom ist und

$R^6$ niederes Alkyl ist und

$C_9$---$C_{10}$ eine CC-Einfach-oder CC-Doppelbindung bedeutet, sowie deren Säure additionssalze.

Bedeutet $R^3$ ein Bromatom, so steht dieses in 12- oder 13-Stellung.

Stellt $C_9$---$C_{10}$ eine CC-Einfachbindung dar, so steht das Wasserstoffatom in 10-Stellung $\alpha$-ständig.

Unter niederen Alkylresten versteht man solche mit bis zu 6 Kohlenstoffatomen, wobei $C_{1-4}$-Alkyle bevorzugt sind wie zum Beispiel Methyl, Ethyl, n-Propyl, Isopropyl, n-Butyl, Isobutyl und tert. Butyl.

Unter einer nucleophilen Gruppe sind bevorzugt die folgenden Reste gemeint: $NR^4R^5$, wobei $R^4$ und $R^5$ jeweils niederes Alkyl ist, $OR^7$, wobei $R^7$ niederes Alkyl oder Acyl ist, $SR^8$, wobei $R^8$ niederes Alkyl oder gegebenenfalls substituiertes Aryl ist, $CHR^9COR^{10}$, wobei $R^9$ $COCH_3$ oder COO-nieder Alkyl bedeutet und $R^{10}$ Wasserstoff, nieder Alkyl oder 0-nieder Alkyl darstellt, $C{\equiv}N$ oder $C{\equiv}CH$.

Der Aromat kann in jeder beliebigen Position ein- oder mehrfach substituiert sein, beispielsweise mit $C_{1-2}$-Alkyl, $C_{1-2}$-Alkoxy, Halogen wie Fluor, Chlor, Brom oder Jod.

Unter Acyl sind bevorzugt Säuren mit bis zu 4 Kohlenstoffatomen zu verstehen wie beispielsweise Essigsäure, Propionsäure, Buttersäure u.a..

Die Salze der erfindungsgemäßen Verbindungen der Formel I sind Säureadditionssalze und leiten sich von üblicherweise verwendeten Säuren ab. Solche Säuren sind zum Beispiel anorganische Säuren, wie beispielsweise Chlorwasserstoffsäure, Salpetersäure, Phosphorsäure, Schwefelsäure, Bromwasserstoffsäure, Jodwasserstoffsäure, salpetrige Säure oder phosphorige Säure, oder organische Säuren, wie beispielsweise aliphatische Mono- oder Dicarbonsäuren, phenylsubstituierte Alkancarbonsäuren, Hydroxyalkancarbonsäuren oder Alkendicarbonsäuren, aromatische Säuren oder aliphatische oder aromatische Sulfonsäuren. Physiologisch unbedenkliche Salze dieser Säuren sind daher z.B. das Sulfat, Pyrosulfat, Bisulfat, Sulfit, Bisulfit, Nitrat, Phosphat, Monohydrogenphosphat, Dihydrogenphosphat, Metaphosphat, Pyrophosphat, Chlorid, Bromid, Jodid, Fluorid, Acetat, Propionat, Decanoat, Caprylat, Acrylat, Formiat, Isobutyrat, Caproat, Heptanoat, Propiolat, Malonat, Succinat, Suberat, Sebacat, Fumarat, Maleat, Mandelat, Butin-1.4-dioat, Hexin-1.6-dioat, Benzoat, Chlorbenzoat, Methylbenzoat, Dinitrobenzoat, Hydroxybenzoat, Methoxybenzoat, Phthalat, Terephthalat, Benzolsulfonat, Toluolsulfonat, Chlorbenzolsulfonat, Xylolsulfonat, Phenylacetat, Phenylpropionat, Phenylbutyrat, Citrat, Lactat, $\beta$-Hydroxybutyrat, Glycollat, Malat, Tartrat, Methansulfonat, Propansulfonat, Naphthalin-1-sulfonat oder Naphthalin-2-sulfonat.

Im Vergleich zu bekannten, in 2-Stellung nicht substituierten Ergolinen, wie zum Beispiel dem Lisurid oder dem Tergurid, zeichnen sich die erfindungsgemäßen Verbindungen der Formel I durch eine zentral antidopaminerge Wirksamkeit und/oder$\alpha_2$-rezeptorblockierende Wirkung aus.

Die zentrale Dopaminrezeptorblockade wurde in einem Interaktionstest mit dem Dopaminrezeptoragonisten Apomorphin an Mäusen nach einmaliger i.p-Vorbehandlung dargestellt (Parameter: Aufhebung der durch Apomorphin 5 mg/kg i.p. verursachten Hypothermie). Männliche NMRI-Mäuse wurden mit verschiedenen Dosen der Substanz, die selbst nicht die Thermoregulation der Versuchstiere beeinflußen, bzw. mit Trägermedium vorbehandelt. 30 Minuten später erhielten alle Tiere Apomorphin 5 mg/kg i.p. 60 Minuten nach Zugabe von Substanz bzw. Trägermedium (= 30 Minuten nach Apomorphin) wurde die Rektaltemperatur mit Hilfe einer Thermosonde gemessen. Während die mit Trägermedium vorbehandelten Mäuse eine Hypothermie aufwiesen, war an mit Substanz vorbehandelten Tieren der körpertemperatursenkende Effekt des Apomorphin dosisabhängig aufgehoben.

Die zentrale $\alpha_2$-Rezeptorblockade wurde in einem Interaktionstest mit dem$\alpha_2$-Rezeptoragonisten Clonidin aus Mäusen nach einmaliger i.p.-Vorbehandlung dargestellt (Parameter: Aufhebung der durch Clonidin 0,1 mg/kg i.p. verursachten Hypothermie). Männliche NMRI-Mäuse wurden mit verschiedenen Dosen der Substanz, die selbst nicht die Thermoregulation der Versuchstiere beeinflussen. bzw. mit Trägermedium vorbehandelt. 30 Minuten später erhielten alle Tiere Clonidin 0,1 mg/kg i.p., 60 Minuten nach Zugabe von Substanz bzw. Trägermedium (=30 Minuten nach Clonidin) wurde die Rektaltemperatur mit Hilfe einer Thermosonde gemessen. Während die mit Trägermedium vorbehandelten Mäuse eine Hypothermie aufwiesen, war an mit Substanz vorbehandelten Tieren der körpertemperatursenkende Effekt des Clondidin dosisabhängig aufgehoben.

Aufgrund dieser Befunde können die erfindungsgemäßen Verbindungen daher als Neuroleptika zur Behandlung von Psychosen des schizophrenen Formenkreises oder als Antidepressiva verwendet werden.

Zur Verwendung der erfindungsgemäßen Verbindungen als Arzneimittel werden diese in die Form eines pharmazeutischen Präparats gebracht, das neben dem Wirkstoff ein für die enterale oder parentale Applikation geeignete pharmazeutische, organische oder anorganische inerte Trägermaterialien, wie zum Beispiel Wasser, Gelatine, Gummi arabicum, Milchzucker, Stärke, Magnesiumstearat, Talk, pflanzliche Öle, Polyalkylenglykole usw. enthält. Die pharmazeutischen Präparate können in fester Form, zum Beispiel als Tabletten, Dragées, Suppositorien, Kapseln oder in flüssiger Form, zum Beispiel als Lösungen, Suspensionen oder Emulsionen vorliegen. Gegebenenfalls enthalten sie darüberhinaus Hilfsstoffe wie Konservierungs,-Stabilisierungs-, Netzmittel oder Emulgatoren, Salze zur Veränderung des osmotischen Drucks oder Puffer.

Die Herstellung der erfindungsgemäßen Verbindungen der allgemeinen Formel I erfolgt nach an sich bekannten Methoden.

Beispielsweise ist das Verfahren zur Herstellung der Verbindungen der allgemeinen Formal I dadurch gekennzeichnet, daß man eine Verbindung der allgemeinen Formel II

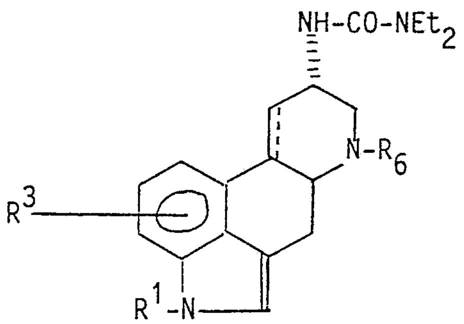 (II),

worin

$R^3$, $R^6$ und $C_9\text{---}C_{10}$ die oben genannte Bedeutung haben und $R^1$ Wasserstoff oder niederes Alkyl darstellt, aminomethyliert, falls $R^1$ Wasserstoff ist, innerhalb von 10 bis 60 Minuten zu einer Verbindung mit $R^1$ in der Bedeutung von $CH_2NR^4R^5$, wobei $R^4$ und $R^5$ die oben genannte Bedeutung haben und gewünschtenfalls diese innerhalb von 10 bis 40 Stunden umwandelt in eine Verbindung mit X in der Bedeutung von $CH_2NR^4R^5$ und $R^1$ in der Bedeutung von $CH_2NR^4R^5$ , Wasserstoff oder $CH_2OH$, wobei $R^4$ und $R^5$ die oben genannte Bedeutung haben und falls $R^1$ niederes Alkyl ist, zu einer Verbindung mit X in der Bedeutung von $CH_2$-$NR^4R^5$ , wobei $R^4$ und $R^5$ die oben genannte Bedeutung haben und gewünschtenfalls anschließend die so erhaltenen Verbindungen gegebenenfalls nach Quartärnisierung mit Methyliodid nucleophil substituiert zu Verbindungen mit $R^2$ in der oben genannten Bedeutung und gegebenenfalls anschließend ein physiologisch verträgliches Säureadditionssalz bildet.

Die Aminomethylierung erfolgt in einer Mannichreaktion mit Formaldehyd und sekundärem Amin in Gegenwart einer Säure oder direkt mit dem Eschenmoser-Salz Dimethyl-methylen-ammonium-halogenid, beispielsweise Jodid oder Chlorid.

Bei der Aminomethylierung erhält man nach einer Reaktionszeit von 10 bis 60 Minuten das kinetische Produkt, das in 1-Stellung substituiert ist. Dieses lagert sich nach einer Reaktionszeit von 10 bis 40 Stunden in Gegenwart von Protonen in das thermodynamisch stabile 2-substituierte Ergolinderivat um. Die Reaktionstemperatur geht von Raumtemperatur bis zu 120 °C.

Falls $R^1$ Wasserstoff ist, werden aus 2-aminomethylierten Ergolinderivaten durch nochmalige Reaktion mit Formaldehyd oder sekundärem Amin in 1-Stellung Verbindungen erhalten, in denen $R^1$ eine $CH_2OH$- oder eine $CH_2NR^4R^5$ -Gruppe darstellt, worin $R^4$ und $R^5$ die oben genannte Bedeutung haben.

Als Säuren sind Mineralsäuren wie HCl u.a. und organische Säuren wie Essigsäure, Propionsäure u.a. geeignet. Die Reaktion kann direkt in der organischen Säure oder in einem polaren protischen oder aprotischen Lösungsmittel durchgeführt werden wie beispielsweise Dimethylacetamid, DMF N-Methylpyrrolidon, Dimethylsulfoxid, Acetonitril, Alkoholen wie Ethanol, Methanol, Propanol, chlorierten Kohlenwasserstoffen wie Methylenchlorid u.a..

Die Aufarbeitung des Reaktionsgemisches erfolgt nach den üblichen Methoden wie beispielsweise durch Kristallisation oder Chromatographie.

Mit oder ohne Isolierung der aminomethylierten Ergolinderivate kann die Dialkylaminogruppe direkt oder nach vorheriger Quartärnisierung nucleophil ausgetauscht werden. Zweckmäßigerweise quartärnisiert man zunächst das Stickstoffatom der Aminomethylengruppe mit Methyliodid in einem inerten Lösungsmittel wie beispielsweise Ethern wie Diisopropylether, Diethylether, Tetrahydrofuran u.a. unter Eiskühlung bis Raumtemperatur in 2 bis 12 Stunden.

Der nucleophile Austausch kann mit oder ohne Isolierung des quartären Ammoniumsalzes vorgenommen werden.

Hierbei wird das nucleophile Anion direkt als Salz beispielsweise als Alkalisalze wie Kalium-, Lithium- oder Natriumsalz verwendet oder es wird durch andere Basen das Anion erzeugt beispielsweise mit Hünig Base, DABCO, DBU, Alkalialkoholate u.a..

Die Reaktion wird unter Eiskühlung bis zu 50 °C durchgeführt und ist im allgemeinen nach 10 bis 40 Mintuen beendet.

Alle inerten Lösungsmittel sind für die nucleophile Substitution geeignet. Beispielsweise genannt seien: Alkohole wie Methanol, Ethanol, Propanol, Butanol, Isopropanol u.a., chlorierte Kohlenwasserstoffe wie Methylenchlorid, Chloroform, Tetrachlorkohlenstoff u.a. acyclische und cyclische Ether wie Diisopropylether, Diethylether, Tetrahydrofuran, Dioxan u.a. Als nucleophile Anionen sind beispielsweise geeignet: Mercaptide, Alkoholate, Amine, Nitrile, Acetylene, β-Dicarbonylverbindungen wie Malonester, Acetessigester, Acetylaceton u.a..

Alle oben beschriebenen Reaktionen werden zweckmäßigerweise unter Inertgasatmosphäre wie Stickstoff oder Argon vorgenommen.

Zur Bildung von Salzen wird die erhaltene Verbindung in wenig Methanol gelöst und mit einer konzentrierten Lösung der gewünschten organischen Säure in Methanol bei Raumtemperatur versetzt.

Die Erfing betrifft ferner die Verwendung von 2-substituierten Ergolinderivaten der allgemeinen Formel I mit X in der Bedeutung von $CH_2$-$NR^4R^5$ zur Herstellung von 2-Methyl-ergolinderivaten der allgemeinen Formel III

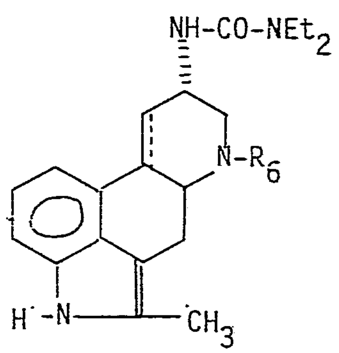

(III),

worin $R^6$ die oben genannte Bedeutung hat. Die erfindungsgemäßen Verbindungen sind daher Zwischenprodukte zur Herstellung von pharmakologisch wirksamen Verbindungen wie sie in der EP-A 0160842 beschrieben sind.

Die Verbindungen der allgemeinen Formel III kann man beispielsweise herstellen, indem man das quartäre Ammo niumsalz der Verbindungen der allgemeinen Formel I mit X in der Bedeutung von $CH_2NR^4R^5$ und $R^1$ und $R^3$ in der Bedeutung von Wasserstoff mit Natriumborhydrid in polaren Lösungsmitteln wie beispielsweise Alkoholen umsetzt zu 2-Methyl-ergolinderivaten. Die Reaktion wird im allgemeinen bei Raumtemperatur durchgeführt und ist nach circa 30 Stunden beendet.

Die Ausgangsverbindungen sind bekannt oder können nach bekannten Methoden hergestellt werden.

Die nachfolgenden Beispiele sollen das erfindungsgemäße Verfahren erläutern.

Beispiel 1

1,1-Diethyl-3-(1-dimethylaminomethyl-6-methyl-8α-ergolinyl)-harnstoff

a) 340 mg Tergurid (1 mMol) löst man in 20 ml Dichlormethan und gibt 240 mg Dimethylmethylenammonium-iodid (1,3 mMol) fest zu. Nach 20 Stunden Rühren bei Raumtemperatur unter Inertgas wird zwischen 1n NaOH und Dichlormethan verteilt, die organische Phase abgetrennt, mit Natriumsulfat getrocknet und eingedampft. Der Rückstand wird an Kieselgel mit Dichlormethan und Methanol chromatographiert und aus Diisoprpylether kristallisiert, Ausbeute 232 mg (58 % d.Th.).

$[\alpha]_D = +8$ ° (0,5 % in Chloroform).

b) Man löst 3,4 g Tergurid in 72 ml Eisessig, gibt 120 ml 40 %ige Dimethylaminlösung und 58 ml 37 % Formaldehydlösung zu und erwärmt 30 Minuten auf 70 ° unter Argon. Die Mischung wird im Eisbad abgekühlt, mit konz. Ammoniak alkalisch gemacht und mit Essigester ausgeschüttelt, Die organische Phase wird mit Natriumsulfat getrocknet, eingedampft und der Rückstand wie oben chromatographiert, Ausbeute 82 %.

1,1,-Diethyl-3-(1-diethylaminomethyl-6-methyl-8α-ergolinyl)-harnstoff dargestellt wie in vorstehendem Beispiel b)

Beispiel 2

1,1-Diethyl-3-(2-dimethylaminomethyl-6-methyl-8α-ergolinyl)-harnstoff

Man löst 596 mg 1,1-Diethyl-3-(1-dimethylaminomethyl-6-methyl-8α-ergolinyl)-harnstoff in 11 ml Eisessig, gibt noch 15 ml 40%ige Dimethylaminlösung und 7 ml 37 %ige Formaldehydlösung zu und rührt über Nacht bei 70 °. Dann wird wie oben beschrieben aufgearbeitet und chromatographiert, wobei man 147 mg isoliert, Aus Diisopropylether kristallisieren 113 mg der gewünschten Verbindung, (19 % d.Th.),
$[\alpha]_D = +\ 14\ °$ (0,5 % in Chloroform)

1,1,Diethyl-3-(2-dimethylaminomethyl-1-hydroxymethyl-6-methyl-8α-ergolinyl)-harnstoff

Eine Fraktion der vorstehend beschriebenen Chromatographie liefert 122 mg, Ausbeute 19 %.

Beispiel 3

1,1-Diethyl-3-(2-diethylaminomethyl-6-methyl-8α-ergolinyl)-harnstoff

In 300 ml Dimethylformamid löst man 10,2 g Tergurid (30 mMol), 33 g Diethylaminhydrochlorid (0,3 Mol) und 7,2 g Paraformaldehyd (0,24 Mol). Man erhitzt 24 Stunden auf 100 ° unter Argon, läßt dann abkühlen und verdünnt mit 100 ml Wasser. Mit 100 ml 1n Natronlauge macht man alkalisch, sättigt die Lösung mit Kochsalz und schüttelt mit Essigester aus. Die organische Phase wird mit Natriumsulfat getrocknet, eingeengt und mehrmals mit Toluol zur Vertreibung des Diemthylformamids destilliert. Der Rückstand wird an Alox (Aktivitätsstufe III) mit Chloroform und Essigester chromatographiert. Von der gewünschten Verbindung werden 8,9 g erhalten (70 %), die aus Essigester kristallisiert werden, Ausbeute 7,1 g (55 % d.Th.)
$[\alpha]_D = +\ 17\ °$ (0,5% in Chloroform).

Als Nebenprodukt wird aus der vorstehenden Chromatographie 3-(1,2-bis-(diethylaminomethyl)-6-methyl-8α-ergolinyl)-1,1-diethyl-harnstoff gewonnen.

Auf gleiche Weise werden die folgenden Mannichbasen dargestellt:
3-(13-Brom-2-diethylaminomethyl-6-methyl-8α-ergolinyl)-1,1-diehtyl-harnstoff Ausbeute 43 %, $[\alpha]_D = -\ 5\ °$ (0,5 % in Chloroform).

3-(9,10-Didehydro-2-diethylaminomethyl-6-methyl-8α-ergolinyl)-1,1-diethylharnstoff
Ausbeute 31 %

1,1-Diethyl-3-(2-diethylaminomethyl)-6-n-propyl-8α-ergolinyl)-harnstoff

1,1-Diethyl-3-(2-diethylaminomethyl-1,6-dimethyl-8α-ergolinyl)-harnstoff

Beispiel 4

(8α-(3,3-Diethylureido)-6-methyl-2-ergolinyl)-diethyl-methyl-ammonium-iodid

2,12 g 1,1 Diethyl-3-(2-diethylaminomethyl-6-methyl-8α-ergolinyl)-harnstoff (5 mMol) löst man in 100 ml Tetrahydrofuran, kühlt im Eisbad und gibt 2,0 ml Iodmethan zu. Man rührt über Nacht bei 3 °C unter Argon, gibt dann 100 ml Diisopropylether zu und filtriert die ausgeschiedenen Kristalle ab. Ausbeute 2,4 g (84 %).
Dieses Quartärsalz wurde ohne weitere Reinigung für die folgenden Umsetzungen verwendet.

Beispiel 5

1,1-Diethyl-3-(2-methoxymethyl-6-methyl-8α-ergolinyl)-harnstoff

345 mg Natrium (15 mMol) werden bei Raumtemperatur unter Argon in 60 ml wasserfreiem Methanol gelöst. Man kühlt im Eisbad ab und gibt 1,7 g Quartärsalz (3 mMol) fest zu. Nach 30 Minuten Rühren bei Raumtemperatur gibt man gesättigte Natriumchloridlösung zu und schüttelt mit Dichlormethan aus. Die organische Phase wird mit Natriumsulfat getrocknet, eingedampft und an Kieselgel mit Dichlormethan und Methanol chromatographiert und aus Diisopropylether kristallisiert, Ausbeute 138 mg (12 %).
$[\alpha]_D = 11\ °$ (0,5 % in Chloroform).

In analoger Weise erhält man mit Natriummethylmercaptid 1,1-Diethyl-3-(6-methyl-2-methylthiomethyl-8α-ergolinyl)-harnstoff

Ausbeute nach Kristallisation aus Dichlormethan und Diisopropylether 12 %, $[\alpha]_D = +\ 18\ °$ (0,5 % in Chloroform).

Mit Natriumphenylmercaptid 1,1-Diethyl-3-(6-methyl-2-phenylthiomethyl-8α-ergolinyl)-harnstoff

Ausbeute nach Kristallisation aus Dichlormethan und Diisopropylether 5 %, $[\alpha]_D = 0\ °$ (0,5 % in Chloroform).

Mit dem Natriumsalz des Acetessigesters 2-Acetyl-3-(8α-(3,3-diethylureido)-6-methyl-2-ergolinyl)-propionsäureethylester

Ausbeute nach Kristallisation aus Diisopropylether 7 %,
$[\alpha]_D = +\ 4\ °$ (0,5 % in Chloroform).

Beispiel 6

1,1-Diethyl-3-(2,6-dimethyl-8α-ergolinyl)-harnstoff

0,8 g Natriumborhydrid (21 mMol) werden in 100 ml Ethanol gelöst. Dazu gibt man unter Argon 2,4 g Quartärsalz fest zu und rührt 23 Stunden bei Raumtemperatur. Zur Vervollständigung der Reaktion werden weitere 0,2 g Natriumborhydrid zugegeben und weitere 7 Stunden gerührt. Man versetzt mit 100 ml Wasser und 100 ml gesättigter Kochsalzlösung, rührt unter Eiskühlung und saugt die Kristalle ab, Ausbeute 0,92 g (52 %),

$[\alpha]_D = + 12\,°$ (0,5 % in Chloroform).

**Patentansprüche**

1.) Verbindungen der allgemeinen Formel I

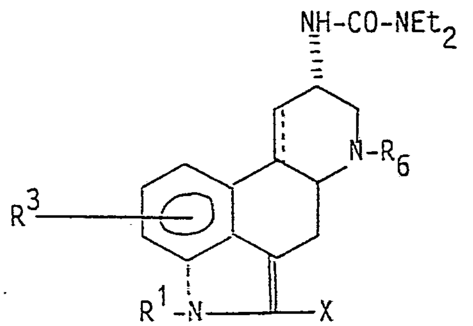

(I),

worin

X eine $CH_2$-$R^2$-Gruppe darstellt mit $R^2$ in der Bedeutung einer nucleophilen Gruppe und

$R^1$ Wasserstoff, niederes Alkyl oder X in der obengenannten Bedeutung ist, und

$R^3$ Wasserstoff oder Brom ist und

$R^6$ niederes Alkyl ist und

$C_9$---$C_{10}$ eine CC-Einfach-oder CC-Doppelbindung bedeutet, sowie deren Säure additionssalze.

2.) 1,1-Diethyl-3-(1-dimethylaminomethyl-6-methyl-8α-ergolinyl)-harnstoff
1,1-Diethyl-3-(1-diethylaminomethyl-6-methyl-8α-ergolinyl)-harnstoff
1,1-Diethyl-3-(2-dimethylaminomethyl-6-methyl-8α-ergolinyl)-harnstoff
1,1-Diethyl-3-(2-dimethylaminomethyl-1-hydroxymethyl-6-methyl-8α-ergolinyl)-harnstoff
1,1-Diethyl-3-(2-diethylaminomethyl-6-methyl-8α-ergolinyl)-harnstoff
3-(1,2-bis-(diethylaminomethyl)-6-methyl-8α-ergolinyl)-1,1-diethyl-harnstoff
3-(13-Brom-2-diethylaminomethyl-6-methyl-8α-ergolinyl)-1,1diethyl-harnstoff
3-(9,10-Didehydro-2-diethylaminomethyl-6-methyl-8α-ergolinyl)-1,1-diethyl-harnstoff
1,1-Diethyl-3-(2-diethylaminomethyl)-6-n-propyl-8α-ergolinyl)-harnstoff
1,1-Diethyl-3-(2-diethylaminomethyl-1,6-dimethyl-8α-ergolinyl)-harnstoff
1,1-Diethyl-3-(2-methoxymethyl-6-methyl-8α-ergolinyl)-harnstoff
1,1-Diethyl-3-(6-methyl-2-methylthiomethyl-8α-ergolinyl)-harnstoff
1,1-Diethyl-3-(6-methyl-2-phenylthiomethyl-8α-ergolinyl)-harnstoff
2-Acetyl-3-(8α-(3,3-diethylureido)-6-methyl-2-ergolinyl)-propionsäure-ethylester

3.) Verfahren zur Herstellung der Verbindungen der allgemeinen Formel I dadurch gekennzeichnet, daß man eine Verbindung der allgemeinen Formel II

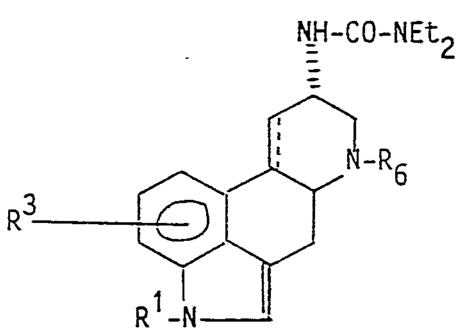

(II),

worin

$R^3$, $R^6$ und $C_9$---$C_{10}$ die oben genannte Bedeutung haben und $R^1$ Wasserstoff oder niederes Alkyl darstellt, aminomethyliert, falls $R^1$ Wasserstoff ist, innerhalb von 10 bis 60 Minuten zu einer Verbindung mit $R^1$ in der Bedeutung von $CH_2NR^4R^5$ , wobei $R^4$ und $R^5$ die oben genannte Bedeutung haben und gewünschtenfalls diese innerhalb von 10 bis 40 Stunden umwandelt in eine Verbindung mit X in der Bedeutung von $CH_2NR^4R^5$ und $R^1$ in der Bedeutung von $CH_2NR^4R^5$ , Wasserstoff oder $CH_2OH$, wobei $R^4$ und $R^5$ die oben genannte Bedeutung haben und falls $R^1$ niederes Alkyl ist, zu einer Verbindung mit X in der Bedeutung von $CH_2$-$NR^4R^5$ , wobei $R^4$ und $R^5$ die oben genannte Bedeutung haben und gewünschtenfalls anschließend die so erhaltenen Verbindungen gegebenenfalls nach Quartärnisierung mit Methyliodid nucleophil substituiert zu Verbindungen mit $R^2$ in der oben genannten Bedeutung und gegebenenfalls anschließend ein physiologisch verträgliches Säureadditionssalz bildet.

4.) Verwendung der Verbindungen der allgemeinen Formel I zur Herstellung von Arzneimitteln.

5.) Verwendung der Verbindungen der allgemeinen Formel Ia

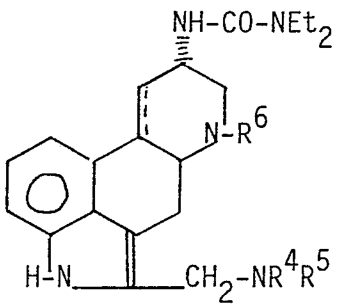

(Ia),

worin $R^4$, $R^5$ und $R^6$ die oben genannte Bedeutung haben zur Herstellung von Verbindungen der allgemeinen Formel III

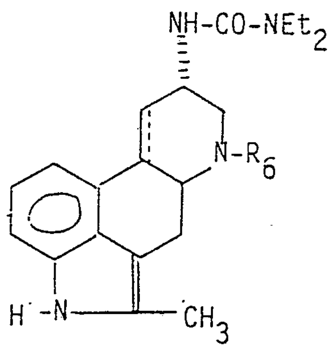

(III),

worin $R^6$ die oben genannte Bedeutung hat.

Europäisches Patentamt

# EUROPÄISCHER RECHERCHENBERICHT

Nummer der Anmeldung

EP 87730064.0

| EINSCHLÄGIGE DOKUMENTE | | | |
|---|---|---|---|
| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
| D,X | EP - A2 - 0 160 842 (SCHERING AKTIENGESELLSCHAFT)<br>* Ansprüche 1,10 *<br>-- | 1,4 | C 07 D 457/12<br>A 61 K 31/48 |
| P,X | EP - A2 - 0 217 736 (SCHERING AKTIENGESELLSCHAFT)<br>* Ansprüche 1,2,4 * | 1,2,4 | |
| P,A | * Anspruch 3c) *<br>-- | 3,5 | |
| A | DE - A1 - 3 308 719 (SPOFA SPOJENE PODNIKY PRO ZDRAVOTNICKOU VYROBU)<br>* Ansprüche 1,9 *<br>-- | 1,4 | |
| A | CH - A - 394 224 (SANDOZ AG)<br>* Anspruch; Seite 2 *<br>-- | 1,4 | RECHERCHIERTE SACHGEBIETE (Int. Cl.4) |
| A | DE - A - 2 335 750 (ELI LILLY AND CO.)<br>* Seite 2; Beispiel 3 *<br>-- | 3,5 | C 07 D 457/00 |
| A | HOUBEN WEYL "Methoden der organischen Chemie" 4. Auflage, Band 11/1, 1957, Georg Thieme Verlag, Stuttgart<br>R. SCHRÖTER "Amine durch Kondensation"<br>Seiten 778-780, 785-793<br>* Seiten 778-780, 788-790, 793 *<br>-- | 3,5 | |

Der vorliegende Recherchenbericht wurde für alle Patentansprüche erstellt.

| Recherchenort | Abschlußdatum der Recherche | Prüfer |
|---|---|---|
| WIEN | 11-08-1987 | PETROUSEK |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

Europäisches Patentamt

# EUROPÄISCHER RECHERCHENBERICHT

Nummer der Anmeldung

EP 87730064.0

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A | HOUBEN-WEYL "Methoden der organischen Chemie" 4. Auflage, Band 11/2, 1958, Georg Thieme Verlag, Stuttgart<br>F. MÜLLER "Eliminierung der Aminogruppe"<br>Seiten 209-221<br>* Seiten 210-213,220 *<br>---- | 3,5 | |

RECHERCHIERTE SACHGEBIETE (Int. Cl 4)

Der vorliegende Recherchenbericht wurde für alle Patentansprüche erstellt.

| Recherchenort | Abschlußdatum der Recherche | Prüfer |
|---|---|---|
| WIEN | 11-08-1987 | PETROUSEK |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument
& : Mitglied der gleichen Patentfamilie, übereinstimmendes Dokument

EPA Form 1503. 03 82